(19) Europäisches Patentamt
European Patent Office
Office européen des brevets

(11) Veröffentlichungsnummer: **0 001 795**
**B1**

(12) # EUROPÄISCHE PATENTSCHRIFT

(21) Anmeldenummer: **78101196.0**

(22) Anmeldetag: **23.10.78**

(51) Int. Cl.³: **C 07 C 69/74,**
A 01 N 37/02,
C 07 C 43/166,
C 07 C 43/174

(54) **Cyclopropankarbonsäure-phenoxy-alpha-vinyl-benzylester, Verfahren zu ihrer Herstellung und ihre Verwendung in der Schädlingsbekämpfung**

(30) Priorität: **01.11.77 CH 13287/77**
**25.09.78 CH 9991/78**

(43) Veröffentlichungstag der Anmeldung:
**16.05.79 Patentblatt 79/10**

(45) Bekanntmachung des Hinweises auf die Patenterteilung:
**07.01.81 Patentblatt 81/01**

(84) Benannte Vertragsstaaten:
**BE CH DE FR GB NL**

(56) Entgegenhaltungen:
**DE - A - 2 635 327**
**DE - A - 2 750 844**
**DE - A - 2 805 226**

(73) Patentinhaber: **CIBA - GEIGY AG**
**Patentabteilung Postfach**
**CH - 4002 Basel (CH)**

(72) Erfinder: **Drabek, Jozef, Dr.**
**Benkenstrasse 12**
**CH - 4104 Oberwil (CH)**
**Ackermann, Peter, Dr.**
**Reichensteinerstrasse 12**
**CH - 4153 Reinach (CH)**
**Farooq, Saleem, Dr.**
**Im Schaiengarten 2**
**CH - 4107 Ettingen (CH)**
**Gsell, Laurenz, Dr.**
**Poolstrasse 26a**
**CH - 4414 Füllinsdorf (CH)**
**Kristiansen, Odd, Dr.**
**Delligrabenstrasse 7**
**CH - 4313 Möhlin (CH)**

**EP 0 001 795 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Cyclopropankarbonsäure-phenoxy-α-vinyl-benzylester, Verfahren zu ihrer Herstellung und ihre Verwendung in der Schädlingsbekämpfung.

Die vorliegende Erfindung betrifft Cyclopropankarbonsäure-phenoxy-α-vinyl-benzylester, Verfahren zu ihrer Herstellung und ihre Verwendung in der Schädlingsbekämpfung.

Die Cyclopropankarbonsäureester haben die Formel

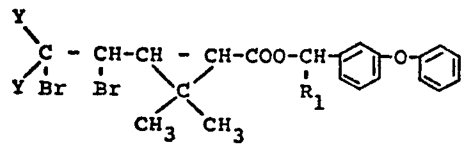

(I)

worin Y Chlor oder Brom und $R_1$ $-CH=CH_2$ oder $-\underset{Br}{\underset{|}{C}}=CHBr$

bedeuten.

Die Verbindungen der Formel I werden nach an sich bekannten Methoden z.B. wie folgt hergestellt:

1)

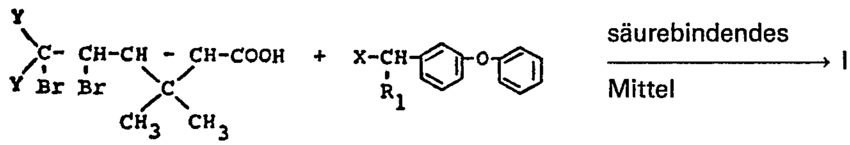


(II) (III)

2)

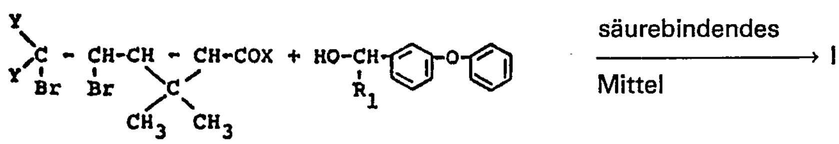


(IV) (V)

3)

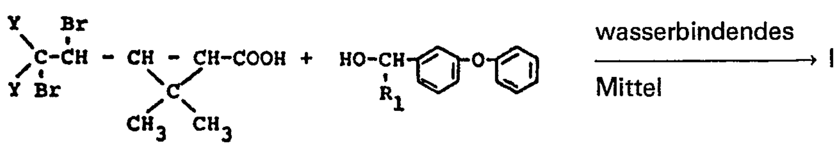


(II) (V)

4)

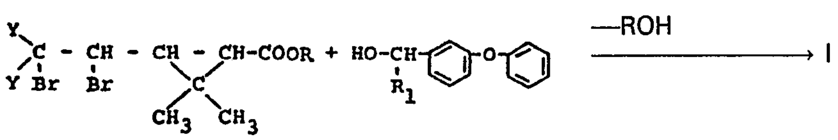


(VI) (V)

In den Formeln II bis VI haben $R_1$ und Y die für die Formel I angegebene Bedeutung.

In den Formeln III und IV steht X für ein Halogenatom, insbesondere Chlor oder Brom und in der Formel VI steht R für $C_1$-$C_4$-Alkyl, insbesondere für Methyl oder Aethyl. Als säurebindendes Mittel für

die Verfahren 1 und 2 kommen insbesondere tertiäre Amine, wie Trialkylamin und Pyridin, ferner Hydroxide, Oxide, Carbonate und Bicarbonate von Alkali- und Erdalkalimetallen sowie Alkalimetallalkoholate wie z.B. Kalium-t.butylat und Natriummethylat in Betracht. Als wasserbindendes Mittel für das Verfahren 3 kann z.B. Dicyclohexylcarbodiimid verwendet werden. Die Verfahren 1 bis 4 werden bei einer Reaktionstemperatur zwischen −10 und 120°C, meist zwischen 20 und 80°C bei normalem oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen wie Diäthyläther, Dipropyläther, Dioxan, Dimethoxyäthan und Tetrahydrofuran; Amide wie N,N-dialkylierte Carbonsäureamide; aliphatische, aromatische sowie halogenierte Kohlenwasserstoffe, insbesondere Benzol, Toluol, Xylol, Chloroform und Chlorbenzol; Nitrile wie Acetonitril; Dimethylsulfoxid und Ketone wie Aceton und Methyläthylketon.

Die Ausgangsstoffe der Formeln II bis VI sind bekannt und können analog bekannten Methoden hergestellt werden.

Die Verbindungen der Formel I liegen als Gemisch von verschiedenen optisch aktiven Isomeren vor, wenn bei der Herstellung nicht einheitlich optisch aktive Ausgangsmaterialien verwendet wurden. Die verschiedenen Isomerengemischen können nach bekannten Methoden in die einzelnen Isomeren aufgetrennt werden. Unter der Verbindung der Formel I versteht man sowohl die einzelnen Isomeren, als auch deren Gemische.

Die Verbindungen der Formel I eignen sich zur Bekämpfung von verschiedenartigen tierischen und pflanzlichen Schädlingen.

Insbesondere eignen sich die Verbindungen der Formel I zur Bekämpfung von Insekten, phytopathogenen Milben und von Zecken z.B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z.B. gegen Spodoptera littoralis und Heliothis virescens) und Gemüsekulturen (z.B. gegen Leptinotarsa decemlineata und Myzus persicae).

Wirkstoffe der Formel I zeigen auch eine sehr günstige Wirkung gegen Fliegen wie z.B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z.B. org.Phosphorverbindungen; Nitrophenole und deren Derivate; Formamidine; Harnstoffe; andere pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt auf Pyrethroide ausüben. Beispiele solcher Verbindungen sind u.a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-(octylsulfonyl)-propyl)-benzol.

Verbindungen der Formel I können für sich allein oder zusammen mit geeigneten Trägern und/oder Zuschlagstoffen eingesetzt werden. Geeignete Zuschlagstoffe können fest oder flüssig sein und entsprechen den in der Formulierungstechnik üblichen Stoffen wie z.B. natürlichen oder regenerierten Stoffen, Lösungs-, Dispergier-, Netz- Haft-, Verdickungs-, Binde-und/oder Düngemittel.

Die Herstellung erfindungsgemässer Mittel erfolgt in an sich bekannter Weise durch inniges Vermischen und/oder Vermahlen der Wirkstoffe der Formel, I mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden:

Feste Aufarbeitungsformen: Stäubemittel, Streumittel, Granulate (Umhüllungsgranulate, Imprägnierungsgranulate und Homogengranulate);

Flüssige Aufarbeitungsformen:

a) in Wasser dispergierbare Wirkstoffkonzentrate:
Spritzpulver (wettable powders), Pasten, Emulsionen;
b) Lösungen

Der Gehalt an Wirkstoff in den oben beschriebenen Mitteln liegt zwischen 0,1 bis 95%, dabei ist zu erwähnen, dass bei der Applikation aus dem Flugzeug oder mittels anderer geeigneter Applikationsgeräte Konzentrationen bis zu 99,5% oder sogar reiner Wirkstoff eingesetzt werden können. Die Wirkstoffe der Formel I können beispielsweise wie folgt formuliert werden (Teile bedeuten Gewichtsteile):

*Stäubemittel:* Zur Herstellung eines a) 5%igen und b) 2%igen Stäubemittels werden die folgenden Stoffe verwendet:

a) 5 Teile Wirkstoff

95 Teile Talkum;

b) 2 Teile Wirkstoff

1 Teil hochdisperse Kieselsäure

97 Teile Talkum.

Der Wirkstoff wird mit den Trägerstoffen vermischt und vermahlen.

*Granulat:* Zur Herstellung eines 5%igen Granulates werden die folgenden Stoffe verwendet:

5 Teile Wirkstoff

0,25 Teile Epichlorhydrin

0,25 Teile Cetylpolyglykoläther

3,50 Teile Polyäthylenglykol

91 Teile Kaolin (Korngrösse 0,3—0,8 mm).

Die Aktivsubstanz wird mit Epichlorhydrin vemischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum verdampft.

*Spritzpulver:* Zur Herstellung eines a) 40%igen, b) und c) 25%igen, d) 10%igen Spritzpulvers werden folgende Bestandteile verwendet:

a) 40 Teile Wirkstoff

5 Teile Ligninsulfonsäure-Natriumsalz

1 Teil Dibutylnaphthalinsulfonsäure-Natriumsalz

54 Teile Kieselsäure;

b) 25 Teile Wirkstoff

4,5 Teile Calcium-Ligninsulfonat

1,9 Teile Champagne-Kreide/Hydroxyäthylcellulose-Gemisch (1:1)

1,5 Teile Natrium-dibutyl-naphthalinsulfonat

19,5 Teile Kieselsäure

19,5 Teile Champagne-Kreide

28,1 Teile Kaolin;

c) 25 Teile Wirkstoff

2,5 Teile Isooctylphenoxy-polyäthylen-äthanol

1,7 Teil Champagne-Kreide/Hydroxyäthylcellulose-Gemisch (1:1)

8,3 Teile Natriumaluminiumsilikat

16,5 Teile Kieselgur

46 Teile Kaolin;

d) 10 Teile Wirkstoff

3 Teile Gemisch der Natriumsalze von gesättigten Fettalkoholsulfaten

5 Teile Naphthalinsulfonsäure/Formaldehyd-Kondensat

82 Teile Kaolin.

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

*Emulgierbare Konzentrate:* Zur Herstellung eines a) 10%igen b) 25%igen und c) 50%igen emulgierbaren Konzentrates werden folgende Stoffe verwendet.

a) 10 Teile Wirkstoff

3,4 Teile epoxydiertes Pflanzenöl

3,4 Teile eines Kombinationsemulgators, bestehend aus Fettalkoholpolyglykoläther und Alkylarylsulfonat-Calcium-Salz

40 Teile Dimethylformamid

43,2 Teile Xylol;

b) 25 Teile Wirkstoff

2,5 Teile epoxydiertes Pflanzenöl

10 Teile eines Alkylarylsulfonat/Fettalkoholpolyglykoläther-Gemisches

5 Teile Dimethylformamid

57,5 Teile Xylol;

c) 50 Teile Wirkstoff

4,2 Teile Tributylphenol-Polyglykoläther

5,8 Teile Calcium-Dodecylbenzolsulfonat

20 Teile Cyclohexanon

20 Teile Xylol.

Aus solchen Konzentration können durch Verdünnen mit Wasser Emulsionen jeder gewünschten Konzentration hergestellt werden.

*Sprühmittel:* Zur Herstellung eines a) 5%igen und b) 95%igen Sprühmittels werden die folgenden Bestandteile verwendet:

a) 5 Teile Wirkstoff

1 Teil Epichlorhydrin

94 Teile Benzin (Siedegrenzen 160—190°C);

b) 95 Teile Wirkstoff

5 Teile Epichlorhydrin.

Beispiel 1

Herstellung von 2,2-Dimethyl-3-(1-brom-2,2-dichlor-2-brom-äthyl)-cyclopropankarbonsäure-3-(phenoxy)-$\alpha$-vinyl-benzylester

Bei 5°C werden zu 8 g 3-Phenoxy-$\alpha$-vinyl-benzylalkohol in 30 ml Benzol unter Rühren 3,2 g Pyridin in 10 ml Benzol zugetropft. Nach der Zugabe von 14 g 2,2-Dimethyl-3(1-brom-2,2-dichlor-2-bromäthyl)-cyclopropankarbonsäurechlorid bei 10°C rührt man das Gemisch 2 Stunden bei Raumtemperatur und lässt es bei dieser Temperatur noch 10 Stunden stehen. Man verdünnt das Reaktionsgemisch mit Eiswasser, extrahiert die organische Schicht 3 x mit je 100 ml 3% HCl und nachher 3 x mit je 100 ml 3% Natriumbikarbonat, trocknet die organische Phase mit Natriumsulfat und destilliert das Benzol ab.

Man erhält die Verbindung der Formel

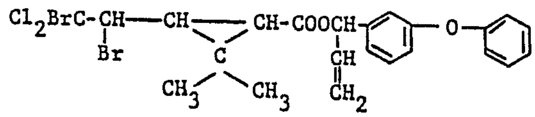

als farbloses Oel mit einer Refraktion von $n_D^{40°} = 1{,}5776$.

Auf analoge Weise erhält man auch die Verbindung der Formel

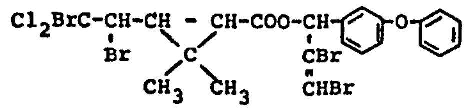

$n_D^{40°} = 1{,}5979$

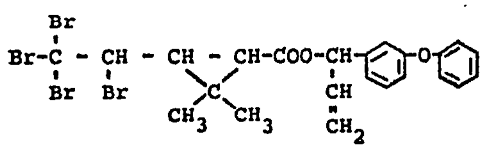

$n_D^{40°} = 1{,}5893$

Beispiel 2

A) Insektizide Frassgift-Wirkung

Baumwollpflanzen wurden mit einer 0,05%igen wässrigen Wirkstoffemulsion (erhalten aus einem 10%igen emulgierbaren Konzentrat) besprüht.

Nach dem Antrocknen des Belages wurden die Baumwollpflanzen je mit Spodoptera littoralis- und Heliothis virescens- Larven $L_3$ besetzt. Der Versuch wurde bei 24°C und 60% relativer Luftfeuchtigkeit durchgeführt.

Verbindungen gemäss Beispiel 1 zeigten im obigen Test eine gute insektizide Frassgift-Wirkung gegen Spodoptera- und Heliothis-Larven.

Beispiel 3

Akarizide Wirkung

Phaseolus vulgaris Pflanzen wurden 12 Stunden vor dem Test auf akarizide Wirkung mit einem infestierten Blattstück aus einer Massenzucht von Tetranychus urticae belegt. Die übergelaufenen beweglichen Stadien wurden aus einem Chromatographiezerstäuber mit den emulgierten Testpräparaten derart besprüht, dass kein Ablaufen der Spritzbrühe eintrat. Nach zwei und 7 Tagen wurden Larven, Adulte und Eier unter dem Binokular auf lebende und tote Individulen ausgewertet und das Ergebnis in Prozenten ausgedrückt. Während der "Haltezeit" standen die behandelten Pflanzen in Gewächshauskabinen bei 25°C.

Verbindungen gemäss Beispiel 1 wirkten im obigen Test gegen Adulte, Larven und Eier von Tetranychus urticae.

Beispiel 4

Wirkung gegen Zecken

A) Rhipicephalus bursa

Je 5 adulte Zecken bzw. 50 Zeckenlarven wurden in ein Glasröhrchen gezählt und für 1 bis 2 Minuten in 2 ml einer wässrigen Emulsion aus einer Verdünnungsreihe mit je 100, 10, 1 oder 0,1 ppm Testsubstanz getaucht. Das Röhrchen wurde dann mit einem genormten Wattebausch verschlossen und auf den Kopf gestellt, damit die Wirkstoffemulsion von der Watte aufgenommen werden konnte.

Die Auswertung erfolgte bei den Adulten nach 2 Wochen und bei den Larven nach 2 Tagen. Für jeden Versuch liefen 2 Wiederholungen.

B) Boophilus microplus (Larven)

Mit einer analogen Verdünnungsreihe wie beim Test A) wurden mit je 20 sensiblen resp. OPresistenten Larven Versuche durchgeführt. (Die Resistenz bezieht sich auf die Verträglichkeit von Diazinon). Verbindungen gemäss Beispiel 1 wirkten in diesen Tests gegen Adulte und Larven von Rhipicephalus bursa und sensible resp. OP-resistente Larven von Boophilus microplus.

**Patentansprüche**

1. Eine Verbindung der Formel

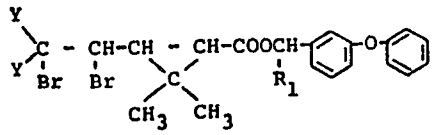

worin Y Chlor oder Brom und

$R_1$ —CH=CH$_2$ oder —C=CHBr
                                   |
                                  Br

bedeuten.

2. Die Verbindung gemäss Anspruch 1 der Formel

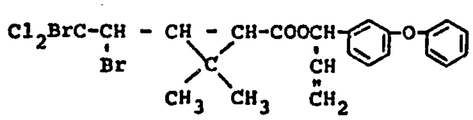

3. Die Verbindung gemäss Anspruch 1 der Formel

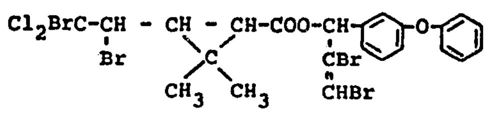

4. Verfahren zur Herstellung von Verbindungen gemäss Anspruch 1, dadurch gekennzeichnet, dass man eine Verbindung der Formel

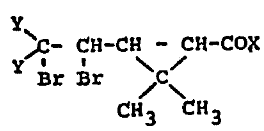

in Gegenwart eines säurebindenden Mittels mit einer Verbindung der Formel

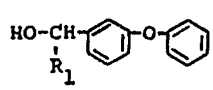

umsetzt,
worin Y und $R_1$ die im Anspruch 1 angegebene Bedeutung haben und X für ein Halogen steht.

5. Ein Schädlingsbekämpfungsmittel, welches als aktive Komponente eine Verbindung gemäss Anspruch 1 mit geeigneten Trägern und/oder andern Zuschlagstoffen.

6. Verwendung einer Verbindung gemäss Anspruch 1 zur Bekämpfung von verschiedenartigen tierischen und pflanzlichen Schädlingen.

7. Verwendung gemäss Anspruch 1 zur Bekämpfung von insekten und Vertreten der Ordnung Akarina.

**Revendications**

1. Un composé répondant à la formule:

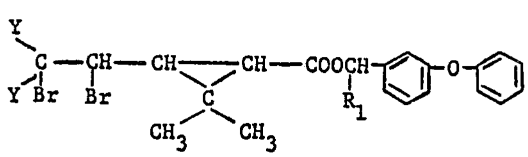

dans laquelle Y représente le chlore ou le brome et

$R_1$ représente —$CH=CH_2$ ou —C=CHBr.
|
Br

2. Le composé selon la revendication 1, rèpondant à la formule:

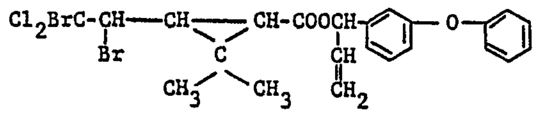

3. Le composé selon la revendication 1, répondant à la formule:

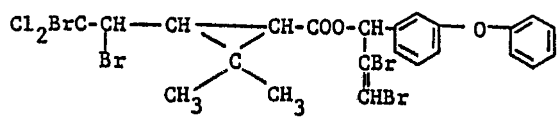

4. Procédé de préparation de composés selon la revendication 1, caractérisé en ce que l'on fait réagir un composé répondant à la formule:

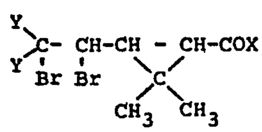

en présence d'un agent neutralisant les acides, avec un composé répondant à la formule:

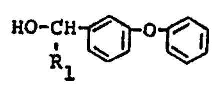

Y et $R_1$ ayant les significations indiquées dans la revendication 1 et X représentant un halogèn

5. Un agent du lutte contre les parasites contenant en tant que composant actif un composé selon la revendication 1 avec des véhicules et/ou autres additifs appropriés.

6. Utilisation d'un composé selon la revendication 1 pour la lutte contre les parasites animaux et végétaux de types variés.

7. Utilisation selon la revendication 6 dans la lutte contre les insectes et les représentants de l'ordre des acariens.

**Claims**

1. A compound of the formula

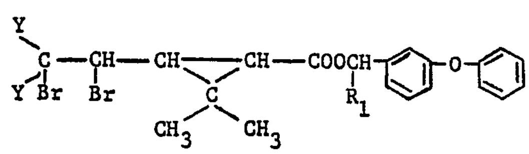

in which Y is chlorine or bromine, and $R_1$ is —$CH=CH_2$ or —C=CHBr.
|
Br

2. The compound according to Claim 1 of the formula

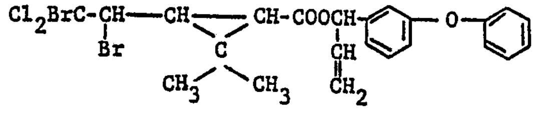

3. The compound according to Claim 1 of the formula

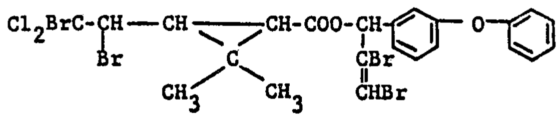

4. A process for producing compounds according to Claim 1, characterised in that a compound of the formula

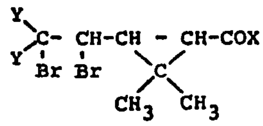

is reacted, in the presence of an acid-binding agent, with a compound of the formula

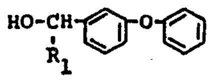

in which Y and $R_1$ have the meanings given in Claim 1, and X is halogen.

5. A pesticidal composition which comprises a compound according to Claim 1 as active ingredient, and suitable carriers and/or other additives.

6. Use of a compound according to Claim 1 for combating various animal and plant pests.

7. Use according to Claim 1 for combating insects, and members of the order *Acarina*.